(19) Europäisches Patentamt
European Patent Office
Office européen des brevets

(11) Veröffentlichungsnummer: **0 394 853 A1**

# (12) EUROPÄISCHE PATENTANMELDUNG

(21) Anmeldenummer: **90107491.4**

(51) Int. Cl.5: **C07K 5/06, A61K 37/64**

(22) Anmeldetag: **19.04.90**

(30) Priorität: **22.04.89 DE 3913272**

(43) Veröffentlichungstag der Anmeldung:
**31.10.90 Patentblatt 90/44**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Nickel, Wolf-Ulrich, Dr.**
**Robert-Stolz-Strasse 120**
**D-6232 Bad Soden am Taunus(DE)**
Erfinder: **Linz, Wolfgang, Dr.**
**Gundelhardtstrasse 2**
**D-6233 Kelkheim (Taunus)(DE)**
Erfinder: **Ruppert, Dieter, Dr.**
**Schreyerstrasse 30**
**D-6242 Kronberg/Taunus(DE)**
Erfinder: **Urbach, Hansjörg, Dr.**
**Le Lavandoustrasse 41**
**D-6242 Kronberg/Taunus(DE)**
Erfinder: **Wagner, Adalbert, Dr.**
**Kleiststrasse 9**
**D-6238 Hofheim am Taunus(DE)**

## (54) Dipeptid-Derivate mit enzym-inhibitorischer Wirkung.

(57) Die vorliegende Erfindung betrifft Verbindungen der Formel I

$$R^a\text{-}W\text{-}A\text{-}B\text{-}NH\text{-}\overset{\displaystyle R^2}{\underset{|}{}}\!\!CH\text{-}\overset{\displaystyle OH}{\underset{|}{}}\!\!CH\text{-}\overset{\displaystyle R^3}{\underset{|}{}}\!\!CH\text{-}R^4 \qquad (I)$$

in welcher A und B unabhängig voneinander eine Aminosäure bedeutet und W, $R^a$, $R^2$, $R^3$ und $R^4$ wie in der Beschreibung angegeben definiert sind, Verfahren zu ihrer Herstellung, ihre Verwendung, sowie diese enthaltende pharmazeutische Zubereitungen.

EP 0 394 853 A1

## Dipeptid-Derivate mit enzym-inhibitorischer Wirkung

Die Erfindung betrifft Dipeptid-Derivate, die die Wirkung des natürlichen Enzyms Renin hemmen sowie Verfahren zu ihrer Herstellung und Verwendung sowie diese enthaltende pharmazeutische Zubereitungen. In der EP-A 0255 082 sind Dipeptid-Derivate beschrieben, die N-terminal Cycloalkylcarbonyl- und Cycloalkyl-Alkylcarbonyl-Substituenten tragen. In der WO 88/05050 sind Aminodiol-Derivate mit renininhibitorischer Wirkung beschrieben. Überraschenderweise wurde nun gefunden, daß substituierte Cycloalkyl- und Cycloalkyl-Alkyl-Derivate zur erheblichen Verbesserung der Wirkung in vivo und zur gesteigerten Resorption der Verbindung beitragen.

Die Erfindung betrifft Verbindungen der Formel I

$$R^a\text{-}W\text{-}A\text{-}B\text{-}NH\text{-}\underset{R^2}{CH}\text{-}\underset{OH}{CH}\text{-}\underset{R^3}{CH}\text{-}R^4 \qquad (I)$$

in welcher

$R^a$ $(C_3$-$C_8)$-Cycloalkyl, $(C_4$-$C_{10})$-Bicycloalkyl, $(C_8$-$C_{12})$-Tricycloalkyl, $(C_3$-$C_8)$-Cycloalkyl-$(C_1$-$C_6)$-Alkyl, $(C_4$-$C_{10})$-Bicycloalkyl-$(C_1$-$C_6)$-alkyl und $(C_8$-$C_{12})$-Tricycloalkyl-$(C_1$-$C_6)$-alkyl bedeutet, worin jeweils der Cycloalkyl-, Bicycloalkyl- und Tricycloalkyl-Substituent durch einen oder zwei gleiche oder verschiedene Reste aus der Reihe F, Cl, Br, I, Hydroxy, $(C_1$-$C_6)$-Alkoxy, $(C_1$-$C_6)$-Alkyl, Carboxy, $(C_1$-$C_6)$-Alkoxycarbonyl, Carbamoyl, Carboxymethoxy, Amino, $(C_1$-$C_6)$-Monoalkylamino, $(C_1$-$C_6)$-Dialkylamino, Amino-$(C_1$-$C_6)$-Alkyl, $(C_1$-$C_6)$-Alkylamino-$(C_1$-$C_6)$Alkyl, Di-$(C_1$-$C_6)$-Alkylamino-$(C_1$-$C_6)$-Alkyl, Amidino, Hydroxamino, Hydroximino, Hydrazono, Imino, Guanidino, $(C_1$-$C_6)$-Alkyloxysulfonyl, $(C_1$-$C_6)$-Alkyloxysulfenyl, Trifluormethyl, $(C_1$-$C_4)$-Alkoxycarbonylamino, $(C_6$-$C_{12})$-Aryl-$(C_1$-$C_4)$-Alkoxycarbonylamino, substituiert sein kann;

W für -CO-, -O-CO-, $-SO_2-$ steht

$R^2$ Wasserstoff, $(C_1$-$C_{10})$-Alkyl, $(C_4$-$C_7)$-Cycloalkyl, $(C_4$-$C_7)$-Cycloalkyl-$(C_1$-$C_4)$-Alkyl, $(C_6$-$C_{14})$-Aryl oder $(C_6$-$C_{14})$-Aryl-$(C_1$-$C_4)$Alkyl bedeutet,

$R^3$ Wasserstoff, $(C_1$-$C_{10})$-Alkyl, $(C_6$-$C_{14})$-Aryl, $(C_6$-$C_{14})$-Aryl-$(C_1$-$C_4)$-Alkyl, Hydroxy oder Amino bedeutet,

$R^4$ einen Rest der Formel II bedeutet,

$(CH_2)_m$-$CHR^5$-D (II)

wobei

$R^5$ für Wasserstoff, $(C_1$-$C_7)$-Alkyl, $(C_1$-$C_5)$-Alkoxy, $(C_1$-$C_5)$-Alkylthio, $(C_1$-$C_5)$-Alkylamino, Hydroxy, Azido, Fluor, Chlor, Brom, Jod steht,

D einen Rest Het bedeutet, wobei

Het einen 5- bis 7-gliedrigen heterocyclischen Ring steht, der benzannelliert, aromatisch, teilhydriert oder vollständig hydriert sein kann, der als Heteroatome einen oder zwei gleiche oder verschiedene Reste aus der Gruppe N, O, S, NO, SO, $SO_2$ enthalten kann und der durch einen oder zwei gleiche oder verschiedene Reste aus der Gruppe $(C_1$-$C_4)$-Alkyl, $(C_1$-$C_4)$-Alkoxy, Hydroxy, Halogen, Amino, Mono- oder Di-$(C_1$-$C_4)$-alkylamino, $CF_3$ substituiert sein kann, und

m 0, 1, 2, 3 oder 4 bedeutet;

A und B unabhängig voneinander einen N-terminal mit $R^a$-W bzw. A und C-terminal mit B bzw. NH-$CHR^2$-CHOH-$CHR^3$-$R^4$ verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo [b]thienylalanin, 2-Fluorphenylalanin, 3-Fluorphenylalanin,4-Pyridylalanin 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, N-Methyl-Histidin, 2-Amino-4-(2-thienyl)-buttersäure, 2-Amino-4-(3-thienyl) buttersäure, 3-(2-Thienyl)-serin, (Z)-Dehydrophenylalanin, (E)-Dehydrophenylalanin, 1,3-Dioxolan-2-yl-alanin, N-Pyrrolylalanin und 1-, 3-oder 4-Pyrazolylalanin bedeuten,

sowie deren physiologisch verträgliche Salze.

Die Chiralitätszentren in den Verbindungen der Formel I können die R-, S- oder R-S-Konfiguration aufweisen.

Alkyl kann geradkettig oder verzweigt sein. Entsprechendes gilt für davon abgeleitete Reste, wie z.B.

Alkoxy, Alkylthio, Alkylamino, Dialkylamino, Alkanoyl und Aralkyl. Unter $(C_3-C_8)$-Cycloalkyl werden Cyclopropyl, Cyclobutyl, Cyclopentyl, Cyclohexyl, Cycloheptyl und Cyclooctyl verstanden.
Unter $(C_4-C_{10})$-Bicycloalkyl bzw. $(C_8-C_{12})$-Tricycloalkyl versteht man einen isocyclischen aliphatischen, nicht aromatischen Rest, der gegebenenfalls unsymmetrisch verteilte Doppelbindungen enthalten kann, gegebenenfalls auch mit offenkettigen aliphatischen Seitenketten substituiert sein kann. Die zwei oder drei Ringe als Komponenten eines derartigen Restes sind kondensiert oder spiroverknüpft und über ein Ring-C-Atom oder ein Seitenketten-C-Atom verknüpft. Beispiele für diese Reste sind Bornyl-, Norbornyl-, Pinanyl-, Norpinanyl-, Caranyl-, Norcaranyl-, Thujanyl-, Adamantyl-, Bicyclo(3.3.0)octyl-, Bicyclo(1.1.0)butyl- , Spiro-(3.3)h eptyl-Substituenten.

Falls die genannten Cyclen mehr als einen Substituenten tragen, so können diese sowohl cis als trans zueinander stehen.

$(C_6-C_{14})$-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Unter Aralkyl versteht man einen mit $(C_1-C_6)$-Alkyl verknüpften unsubstituierten oder substituierten $(C_6-C_{14})$-Aryl-Rest, wie z.B. Benzyl, $\alpha$- und $\beta$-Naphthylmethyl, Halobenzyl und Alkoxybenzyl, wobei Aralkyl jedoch nicht auf die genannten Reste beschränkt wäre.

Ein Rest Het im Sinne vorstehender Definition ist beispielsweise Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, $\beta$-carbolinyl oder ein benzannelliertes, cyclopenta-, cyclohexa- oder cyclohepta-annelliertes Derivat dieser Reste. Dieser Heterocyclus kann an einem Stickstoffatom durch Oxido, $(C_1-C_6)$-Alkyl, z.B. Methyl oder Ethyl, Phenyl oder Phenyl-$(C_1-C_4)$-alkyl, z.B. Benzyl, und/oder an einem oder mehreren Kohlenstoffatomen durch $(C_1-C_4)$-Alkyl, z.B. Methyl, Phenyl, Phenyl-$(C_1-C_4)$-alkyl, z.B. Benzyl, Halogen, z.B. Chlor, Hydroxy, $(C_1-C_4)$-Alkoxy, z.B. Methoxy, Phenyl-$(C_1-C_4)$-alkoxy, z.B. Benzyloxy, oder Oxo substituiert und teilweise gesättigt sein und ist beispielsweise 2- oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3-oder 4-pyridino, 2-Pyrazinyl, 2-, 4- oder 5-Pyrimidinyl, 2-, 3- oder 5-Indolyl, substituiertes 2-Indolyl, z.B. 1-Methyl-, 5-Methyl-, 5-Methoxy, 5-Benzyloxy-, 5-Chlor-oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl oder $\beta$-Carbolin-3-yl.

Teilhydrierte oder vollständig hydrierte heterocyclische Ringe sind beispielsweise Dihydropyridinyl, Pyrrolidinyl, z.B. 2-, 3- oder 4-N-Methylpyrrolidinyl, Piperidinyl, Piperazinyl, Morpholino, Thiomorpholino, Tetrahydrothiophenyl.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in welcher
$R^a$ und W wie auf Seite 1 definiert sind;
$R^2$ Isobutyl, Benzyl oder Cyclohexylmethyl bedeutet;
$R^3$ Wasserstoff, $(C_1-C_5)$-Alkyl, $(C_6-C_{10})$-Aryl, $(C_6-C_{10})$-Aryl-$(C_1-C_4)$-alkyl oder Hydroxy bedeutet;
$R^4$ einen Rest der Formel II bedeutet, worin
$R^5$ für Wasserstoff, $(C_1-C_4)$-Alkyl, $(C_1-C_4)$-Alkoxy, $(C_1-C_4)$-Alkylthio, $(C_1-C_4)$-Alkylamino, Hydroxy, Azido, Fluor, Chlor, Brom oder Jod steht,
D wie der Rest Het auf Seite 2 definiert ist, und
m 0, 1 oder 2 bedeutet; und
A und B unabhängig voneinander einen bivalenten Rest aus der Reihe
Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furylalanin, $\beta$-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin,4-Pyridylalanin 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, Cystein, S-Methyl-Cystein, N-Methyl-Histidin, 1,2,3,4-Tetrahydroisochinolin-3-carbonsäure, Homophenylalanin, 2-Amino-4-(2-thienyl)-buttersäure, 2-Amino-4-(3-thienyl)-buttersäure, 3-(2- Thienyl)-serin, (Z)-Dehydrophenylalanin, (E)-Dehydrophenylalanin, 1,3-Dioxolan-2-yl-alanin, N-Pyrrolylalanin und 1-, 3-oder 4-Pyrazolylalanin, bedeuten,
sowie deren physiologisch verträgliche Salze.

Insbesondere bevorzugt sind Verbindungen der Formel I, in welcher
$R^a$ ($C_4$-$C_6$)-Cycloalkyl und ($C_4$-$C_6$)-Cylcoalkyl-($C_1$-$C_2$)-Alkyl bedeutet, worin jeweils der Cycloalkyl-Substituent durch einen oder zwei gleiche oder verschiedene Reste aus der Reihe Hydroxy, ($C_1$-$C_4$)-Alkoxy, ($C_1$-$C_2$)-Alkyl, Carboxy, ($C_1$-$C_2$)-Alkoxycarbonyl,Carbamoyl, Carboxymethoxy, Amino, ($C_1$-$C_2$)-Monoalkylamino, ($C_1$-$C_2$)-Dialkylamino, Amino-($C_1$-$C_2$)-Alkyl, ($C_1$-$C_2$)-Alkylamino-($C_1$-$C_2$)Alkyl, Di-($C_1$-$C_2$)-Alkylamino-($C_1$-$C_2$)-Alkyl, Amidino, Trifluormethyl, ($C_1$-$C_4$)-Alkoxycarbonylamino, wie tert.Butoxycarbonylamino, ($C_6$-$C_{12}$)-Aryl-($C_1$-$C_2$)-Alkoxy-Carbonylamino wie Benzyloxycarbonylamino und Methansulfonylamido, Trifluormethansulfonylamido substituiert sein kann;
W für -CO- steht
$R^2$ Isobutyl, Benzyl oder Cyclohexylmethyl bedeutet;
$R^3$ Wasserstoff oder Hydroxy bedeutet;
$R^4$ einen Rest der Formel II bedeutet, in welcher
$R^5$ für Wasserstoff oder Fluor steht,
D für einen 2-, 3- oder 4-Pyridinrest, einen 2-, 4-oder 5-Imidazolrest oder einen 2-Oxazolinrest steht, wobei die genannten Heterocyclen jeweils durch einen oder zwei Reste aus der Gruppe Methyl,Ethyl, Propyl, Allyl, Fluor, Chlor, Brom, $CF_3$ und Methoxy substituiert sein können, und
m 0, 1 oder 2 bedeutet; und
A und B unabhängig voneinander einen bivalenten Rest aus der Reihe
Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin,4-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, 1,2,3,4-Tetrahydroisochinolin-3-carbonsäure, Homophenylalanin, 2-Amino-4-(2-thienyl)-buttersäure und 1-, 3- und 4-Pyrazolylalanin bedeuten,
sowie deren physiologisch verträgliche Salze.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln IIIa, IIIb, IIIc:

$R^a$-W-OH      $R^a$-W-A-OH      $R^a$-W-A-B-OH

IIIa      IIIb      IIIc

Fragmente einer Verbindung der Formel I mit einer endständigen Aminogruppe besitzen die nachstehenden Formeln IVa, IVb, IVc:

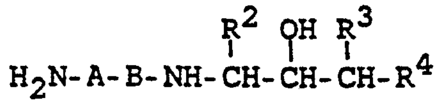

IVa

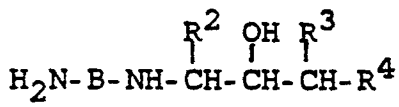

IVb

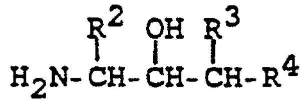

IVc

Methoden, die zur Herstellung einer Amidbindung geeignet sind, werden z.B. in Houben-Weyl, Methoden der organischen Chemie, Band 15/2; Bodanszky et al., Peptide synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen: Aktivestermethode mit N-Hydroxy-succinimid oder 1-Hydroxybenzotrizol als Esterkomponente, Kupplung mit einem Carbodiimid wie Dicyclohexylcarbodiimid oder mit Propanphosphonsäureanhydrid und die Gemischt-Anhydrid-Methode mit Pivaloylchlorid.

Die Herstellung der als Ausgangsverbindungen verwendeten optisch aktiven Amine der Formel IVc

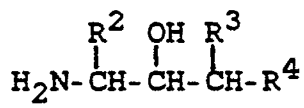

IVc

worin $R^2$, $R^3$ und $R^4$ wie oben definiert sind, erfolgt ausgehend von optisch aktiven $\alpha$-Aminosäuren, wobei deren Asymmetriezentrum erhalten bleibt. Hierzu wird in bekannter Weise ein N-geschützter Aminosäurealdehyd hergestellt, welcher in einer Aldol-analogen Addition an einen entsprechenden Heteroarylalkyl-Baustein gekuppelt wird und nach Abspaltung der N-Schutzgruppe Aminoalkohole der Formel IVc ergibt. Man erhält Diastereomerengemische bezüglich des OH-tragenden Zentrums, die in an sich bekannter Weise, beispielsweise durch fraktionierte Kristallisation oder durch Chromatographie getrennt werden. Die Überprüfung der Diastereomerenreinheit erfolgt mittels HPLC, die Enantiomerenreinheit kann in bekannter Weise durch Überführung in Mosher-Derivate überprüft werden (H.S. Mosher et. al., J. org. Chem. **34**, 2543 (1969)).

Die Herstellung N-geschützter Aminosäurealdehyde erfolgt nach B. Castro et al. (Synthesis 1983, 676).

Die Aldol-analoge Addition an N-geschützte Aminosäurealdehyde (bevorzugterweise N-tert.-Butoxycarbonyl-und Benzyloxycarbonyl-Schutzgruppen) erfolgt in einem gegenüber Basen inerten Lösungsmittel, wie Ether, THF, Toluol, DMF, DMSO oder Dimethoxyethan.

Als Basen zur Deprotonierung der Heteroarylalkyl-Komponente können Alkalimetallalkoholate, wie Kalium-O-tert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die zur Herstellung von Verbindungen der Formel I erforderlichen Vor- und Nachoperationen wie Einführung und Abspaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt.

Stereoisomerengemische, insbesondere Diastereomerengemische, die bei Verwendung racemischer Aminosäuren A oder B anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen, welches als Folge verschiedener Stimuli (Volumendepletion, Natriummangel, $\beta$-Rezeptorenstimulation) von den juxtaglomerulären Zellen der Niere in den Blutkreislauf

sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

**1. Testprinzip**

Z.B. Humanplasma, welches sowohl Renin als auch Angiotensinogen enthält, wird bei 37 °C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung wird durch Renin-Inhibitoren gehemmt.

**2. Gewinnung des Plasmas**

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4 °C, 15 min; wiederholen, falls erforderlich) wird das Plasma vorsichtig abpipettiert und in geeigneten Portionen bei -30 °C eingefroren. Für den Test werden nur Plasmen mit ausreichend hoher Reninaktivität verwendet. Plasmen mit niedriger Reninaktivität werden durch eine Kältebehandlung (-4 °C, 3 Tage) aktiviert (Prorenin → Renin).

**3. Durchführung des Tests**

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:
Inkubationsansatz:
1000 µl Plasma (bei 0-4 °C aufgetaut)
100 µl Phosphatpuffer (pH 7,4)
Zusatz von $10^{-4}$ M Ramiprilat)
10 µl PMSF-Lösung
10 µl 0,1 % Genapol PFIC
12 µl DMSO bzw. Testpräparat

Die Testpräparate werden i.a. $10^{-2}$ M in 100 % Dimethylsulfoxid (DMSO) gelöst und mit DMSO entsprechend verdünnt; der Inkubationsansatz enthält max. 1 % DMSO.

Die Ansätze werden in Eis gemischt und für 1 Stunde zur Inkubation in ein Wasserbad (37 °C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25 °C für die Angiotensin I-Bestimmung aufbewahrt (Mittelwert aus drei Einzelproben).

**Angiotensin I-Radioimmunoassay (RIA)**

Es wird exakt die Gebrauchsanweisung des RIA-Kits (Renin-Maia®-kit, Serono Diagnostics S.A.,

Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel I zeigen in dem in-vitro-Test Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1-50 mg/kg. Die in der vorliegenden Erfindung beschriebenen Verbindungen der allgemeinen Formel I können als Antihypertensiva, sowie zur Behandlung der Herzinsuffizienz verwendet werden.

Die HIV-Protease schneidet sich autokatalytisch aus dem GAG-POL-Polypeptid heraus und spaltet anschließend das Vorläuferpeptid p55 in die Core-Antigene p17, p24 und p14. Sie ist damit ein essentielles Enzym deren Inhibition den Lebenszyklus des Virus unterbricht und seine Vermehrung unterbindet.

In biologischen Tests zeigte sich, daß die erfindungsgemäßen Verbindungen enzyminhibitorische Wirkung haben und auch virale Enzyme wie die HIV-Protease hemmen. Besondere Bedeutung hat die HIV-Protease inhibierende Wirking, die die erfindungsgemäßen Verbindungen insbesondere zur Therapie und Prophylaxe von durch Infektion mit HIV bedingten Erkrankungen qualifiziert. Die erfindungsgemäßen Verbindungen der allgemeinen Formel I zeigen in verwendeten in-vitro-Tests Hemmwirkungen bei Konzentrationen von etwa $10^{-4}$ bis $10^{-8}$ Mol/l.

Zum Gegenstand der Erfindung gehört weiterhin die Verwendung von Verbindungen der Formel I zur Herstellung von Arzneimittel zur Bluthochdrucktherapie und der Behandlung der kongestiven Herzinsuffizienz sowie zur Therapie und Prophylaxe von Viruserkrankungen, insbesondere von Erkrankungen, die durch das HIV verursacht werden sowie die genannten Arzneimittel.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage Wasser, physiologische Kochsalzlösungen oder Alkohole, z.B. Ethanol, Propandiol oder Glycerin, daneben auch Zuckerlösungen wie Glucose-oder Mannitlösungen, oder auch eine Mischung aus den verschiedenen genannten Lösungsmitteln.

**Verzeichnis der verwendeten Abkürzungen:**

ACC 4-Aminocyclohexylcarbonyl
Boc tert.-Butoxycarbonyl
BuLi n-Butyl-Lithium
DCC Dicyclohexylcarbodiimid

DCI Desorption Chemical Ionisation
DNP 2,4-Dinitrophenyl
DME Dimethoxyethan
DMF Dimethylformamid
EE Essigsäureethylester
EI Electron Impact
FAB Fast atom bombardment
h Stunde
HOBt 1-Hydroxybenzotriazol
M Molekularpeak
MS Massenspektrum
MTBE Methyl-tert.butylether
NEM N-Ethylmorpholin
n-PropPA n-Propylphosphonsäureanhydrid
THF Tetrahydrofuran

Die sonstigen für Aminosäuren verwendeten Abkürzungen entsprechen dem in der Peptidchemie üblichen Drei-Buchstaben-Code wie er z.B. in Eur. J. Biochem. 138, 9-37 (1984) beschrieben ist. Falls nicht ausdrücklich anders angegeben, handelt es sich immer um Aminosäuren der L-Konfiguration.

Die verwendeten Laufmittel sind folgendermaßen abgekürzt

| | |
|---|---|
| LM1 | $CH_2Cl_2/CH_3OH$ 20:1 |
| LM2 | $CH_2Cl_2/CH_3OH$ 9:1 |
| LM3 | $CH_2Cl_2/CH_3OH$ 12:1 |
| LM4 | $CH_2Cl_2/CH_3OH$/conc. $NH_3$ 10:1:0,1 |
| LM5 | Cyclohexan/Essigester 9:1 |
| LM6 | n-Hexan/Essigester 2:1 |

Die nachstehenden Beispiele dienen zur Erläuterung der vorliegenden Erfindung, ohne daß diese darauf beschränkt wäre.

**Beispiel 1**

N-(N-Tert-butoxycarbonyl-cis-4-aminocyclohexylcarbonyl)-L-phenylalanin-L-norvalin-(1-S-cyclohexylmethyl-2-S-hydroxy-5-(2-pyridyl))-pentylamid

235 mg N-(N-Tert-butoxycarbonyl-cis-4-aminocyclohexylcarbonyl)-L-phenylalanin gelöst in 3 ml DMF wurden mit 98 mg HOBt, 130 mg DCC und 0,08 ml NEM versetzt. Zu dieser Lösung wurde 230 mg L-Norvalin-(1-S-cyclohexylmethyl-2-S-hydroxy-5-(2-pyridyl))-pentylamid, gelöst in 3 ml DMF, gegeben und über Nacht bei RT gerührt.
Das DMF wurde im Hochvakuum abdestilliert, die verbliebene Lösung in Essigester aufgenommen, vom ausgefallenen Harnstoff abfiltriert und zweimal mit $NaHCO_3$ ges. und NaCl-Lösung ges. gewaschen, die organische Phase über $MgSO_4$ getrocknet und i. Vak. eingeengt. Nach Säulenchromatographie (Kieselgel, LM1) wurden 315 mg isoliert.
Schmelzpunkt 100-103 °C
MS (FAB) 748 (M+1)

a) N-(N-Tert-butoxycarbonyl-cis-4-aminocyclohexylcarbonyl)-L-phenylalanin

5,5 g N-(N-Tert-butoxycarbonyl-cis-4-aminocyclohexylcarbonyl)-L-phenylalaninbenzylester wurden in 230 ml Ethanol über 1 g Pd/Kohle unter Normaldruck hydriert. Nach beendeter Reaktion wurde vom Katalysator abfiltriert und das Lösungsmittel abdestilliert. Nach Umkristallisation aus n-Heptan/Essigester wurden 4,1 g farbloses Produkt erhalten.
Schmelzpunkt 160-161 C (Zersetzung)

MS (DCI) 391 (M+1)

b) N-(N-Tert-butoxycarbonyl-cis-4-aminocyclohexylcarbonyl)-L-phenylalaninbenzylester

6,0 g N-Tert-butoxycarbonyl-cis-1,4-aminocyclohexancarbonsäure und 6,3 g L-Phenylalaninbenzylester wurden in 75 ml DMF gelöst und mit 24 ml n-PropylPA und 15,7 ml NEM bei 0°C gemischt und über Nacht bei RT zur Reaktion gebracht. Die Lösung wurde mit $CH_2Cl_2$ verdünnt und jeweils mit halbges. $NaHCO_3$-Lösung, 10 %-Citronensäure und Wasser gewaschen, über $MgSO_4$ getrocknet und i.Vak. eingeengt.
Flashchromatographie ergab 5,7 g reines Produkt.
$\alpha_D$ : -14,6° (c = 1,1 in $CH_3OH$)

**Beispiel 2**

N-(cis-4-Aminocyclohexylcarbonyl)-L-phenylalanin-L-norvalin-(1-S-cyclohexylmethyl-2-S-hydroxy-5-(2-pyridyl))-pentylamid

77,7 mg der in Beispiel 1 beschriebenen Verbindung wurden in 2 ml $CH_2Cl_2$ gelöst und bei 0°C unter $N_2$ mit 2 ml $CF_3COOH$ versetzt. Nach 30 Min. wurde die Lösung eingeengt, der Rückstand in Essigester aufgenommen und jeweils zweimal mit ges. $NaHCO_3$ und NaCl-Lösung gewaschen.
Säulenchromatographie (Kieselgel, LM1) ergab 24,7 mg Produkt.
MS (FAB) 648 (M+1)

**Beispiel 3**

N-(N-Tert-butoxycarbonyl-trans-4-aminocyclohexylcarbonyl)-L-phenylalanin-L-norvalin-(1-S-cyclohexylmethyl-2-S-hydroxy-5-(2-pyridyl))-pentylamid

Analog des in Beispiel 1 beschriebenen Verfahrens wurde die Titelverbindung aus N-(N-Tert-butoxycarbonyl-trans-4-aminocyclohexylcarbonyl)-L-phenylalanin und L-Norvalin-(1-S-cyclohexylmethyl-2-S-hydroxy-5-(2-pyridyl))-pentylamid hergestellt. Die Ausbeute betrug 270 mg.
Schmelzpunkt: 219-220°C.
MS (FAB) 748 (M+1)

a) N-(N-Tert-butoxycarbonyl-trans-4-aminocyclohexylcarbonyl)-L-phenylalanin

4,45 g N-(N-Tert-butoxycarbonyl-trans-4-aminocyclohexylcarbonyl)-L-phenylalaninbenzylester wurden in 200 ml Ethanol bei RT unter Normaldruck über 900 mg Palladium-Kohle katalytisch hydriert. Nach beendeter Reaktion wurde vom Katalysator abfiltriert und das Lösungsmittel im Vakuum abdestilliert. Nach Umkristallisation wurden 2,67 g reines Produkt erhalten.
Schmelzpunkt: ab 160°C Zersetzung
MS (DCI): 391 (M+)

b) N-(N-Tert-butoxycarbonyl-trans-4-aminocyclohexylcarbonyl)-L-phenylalaninbenzylester

3 g N-Tert-butoxycarbonyl-trans-4-aminocyclohexylcarbonsäure und 3,2 g L-Phenylalaninbenzylester wurden in 40 ml DMF gelöst und bei 0°C mit 7,85 ml NEM und anschließend mit 12 ml n-PropPA versetzt. Die Mischung wurde über Nacht bei RT zur Reaktion gebracht. Nach Verdünnen mit 200 ml Essigester wurde die Lösung jeweils zweimal mit $H_2O$, 10 % Citronensäure ges. $NaHCO_3$ und ges. NaCl-Lösung gewaschen. Die verbliebene organische Phase wurde mit $MgSO_4$ getrocknet und im Vakuum vom Lösungsmittel befreit. Das Rohprodukt wurde mit Diethylether verrieben, die ausgefallenen Kristalle abgesaugt und getrocknet. Es wurden 4,45 g farbloser Kristalle erhalten.
Schmelzpunkt: 176-177 °C
MS (DCI) 481 (M+1)

**Beispiel 4**

N-(trans-4-Aminocyclohexylcarbonyl)-L-phenylalanin-L-norvalin-(1-S-cyclohexylmethyl-2-S-hydroxy-5-(2-pyridyl))-pentylamid

Die Titelverbindung wurde nach dem in Beispiel 2 beschriebenen Verfahren aus der in Beispiel 3 beschriebenen Verbindung hergestellt.
Ausbeute nach Chromatographie (Kieselgel, LM2) 27 mg.
MS (FAB) 648 (M+1); $R_F$ 0,17 (Methylenchlorid/Methanol 8:2)

**Beispiel 5**

N-(N-Tert-butoxycarbonyl-trans-4-aminocyclohexylcarbonyl)-L-phenylalanin-N(im)-Trityl-L-histidin-(1-S-cyclohexymethyl-2-S-hydroxy-5-(2-pyridyl))-pentylamid

Die Titelverbindung wurde aus dem in Beispiel 3a) beschriebenen Phenylalanin-Derivat und aus FMOC-His(Trt)-(1-S-cyclohexymethyl-2-S-hydroxy-5-(2-pyridyl))-pentylamid nach dem in Beispiel 1 beschriebenen Verfahren hergestellt.
Ausbeute nach Chromatographie (Kieselgel, LM1) 0,4 g
MS (FAB) 1028 (M+1), 1034 (M+7). $R_F$ 0,5 (Toluol/Ethanol 8:2)

**Beispiel 6**

N-(N-Tert-butoxycarbonyl-trans-4-aminocyclohexylcarbonyl)-L-phenylalanin-L-histidin-(1-S-cyclohexylmethyl-2-S-hydroxy-5-(2-pyridyl))-pentylamid

40 mg der in Beispiel 5 erhaltenen Substanz wurde in 5 ml 90 % Essigsäure gelöst und für zwei Stunden auf 60 °C erhitzt. Die Lösung wurde mit $Na_2CO_3$ auf pH 8 gestellt und dreimal mit Essigester extrahiert. Die organische Phase wurde jeweils zweimal mit ges. $NaHCO_3$-Lösung und mit ges. NaCl-Lösung gewaschen, anschließend über $MgSO_4$ getrocknet und i.Vak. eingeengt. Chromatographie (Kieselgel, LM1) ergab 16 mg reines Produkt.
MS (FAB) 786 (M+1) Zers. ab 155 °C

**Beispiel 7**

N-(trans-4-Aminocyclohexylcarbonyl)-L-phenylalanin-L-histidin-(1-S-cyclohexylmethyl-2-S-hydroxy-5-(2-pyridyl))-pentylamid

300 mg der in Beispiel 5 beschriebenen Substanz wurde mit 5 ml $CF_3COOH$ und 0,25 ml $H_2O$ für 15 Min. bei 0 °C und für 2 h bei RT gerührt. Die Lösung wurde i.Vak. eingeengt mit $NaHCO_3$-Lösung alkalisch gestellt und mit Essigester extrahiert. Die organische Phase wurde i.Vak. eingeengt und der Rückstand chromatographiert (Kieselgel, LM2).
MS (FAB) 686 (M+1). Schmp. 114-116 °C

**Beispiel 8**

N-(N-Tert butoxycarbonyl-cis-4-aminocyclohexylcarbonyl)-L-phenylalanin-N(im)-Trityl-L-histidin-(1-S-cyclohexylmethyl-2-S-hydroxy-5-(2-pyridyl))-pentylamid

Die Titelverbindung wurde aus dem in Beispiel 1a) beschriebenen Phenylalanin-Derivat und aus FMOC-His(Trt)-(1-S-cyclohexylmethyl-2-S-hydroxy-5-(2-pyridyl))-pentylamid nach dem in Beispiel 1 beschriebenen Verfahren hergestellt.
Ausbeute nach Chromatographie (Kieselgel, LM1) 0,5 g.
MS (FAB) 1028 (M+1), 1034 (M+7); $R_F$ 0.26 (LM 1)

**Beispiel 9**

N-(N-Tert-butoxycarbonyl-cis-4-aminocyclohexylcarbonyl)-L-phenylalanin-L-histidin-(1-S-cyclohexylmethyl-2-S-hydroxy-5-(2-pyridyl))-pentylamid

Die Titelverbindung wurde nach dem in Beispiel 6 beschriebenen Verfahren aus der in Beispiel 8 beschriebenen Verbindung hergestellt. Die Ausbeute betrug 61 mg. Schmp. 127-130 °C
MS (FAB) 786 (M+1) ; $R_F$0.21 (LM 2)

**Beispiel 10**

N-(cis-4-Aminocyclohexylcarbonyl)-L-phenylalanin-L-histidin-(1-S-cyclohexylmethyl-2-S-hydroxy-5-(2-pyridyl))-pentylamid

Die Titelverbindung wurde nach dem in Beispiel 7 beschriebenen Verfahren aus der in Beispiel 8 beschriebenen Verbindung hergestellt. Ausbeute 180 mg.
MS (FAB) 686 (M+1).
Schmp. 177 °C Zers.

**Beispiel 11**

N-(N-Tert-butoxycarbonyl-trans-4-aminocyclohexylcarbonyl)-L-tyrosin(o-methyl)-N-(im)-trityl-L-histidin-(1-S-cyclohexylmethyl-2-S-hydroxy-5-(2-Pyridyl))-pentylamid

Die Titelverbindung wurde aus N-(N-Tert-butoxycarbonyl-trans-4-aminocyclohexylcarbonyl)-L-tyrosin-(methyl) und L-His(trityl)-(1-S-Cyclohexylmethyl-2-S-hydroxy-5-(2-pyridyl))-pentylamid nach dem in Beispiel 1 beschriebenen Verfahren hergestellt. $R_F$ 0.54 (Toluol/Ethanol 8:2)
MS (FAB) 1058 (M+1)

**Beispiel 12**

N-(N-Tert-butoxycarbonyl-trans-4-aminocyclohexylcarbonyl)-L-tyrosin(o-methyl)-L-histidin (1-S-cyclohexylmethyl-2-S-hydroxy-5-(2-pyridyl))-pentylamid

Die Titelverbindung wurde nach dem in Beispiel 6 beschriebenen Verfahren aus der in Beispiel 11 beschriebenen Verbindung hergestellt. $R_F$0,2 (Toluol/Ethanol 8:2)
MS (FAB) 816 (M+1) Zers. ab 180 °C

**Beispiel 13**

N-(trans-4-Aminocyclohexylcarbonyl)-L-tyrosin(o-methyl)-L-histidin (1-S-cyclohexylmethyl-2-S-hydroxy-5-(2-pyridyl))-pentylamid

Die Titelverbindung wurde nach dem in Beispiel 7 beschriebenen Verfahren aus der in Beispiel 11 beschriebenen Verbindung hergestellt.
MS (FAB) 716 (M+1)

**Beispiel 14**

N-(N-Tert-butoxycarbonyl-cis-4-aminocyclohexylcarbonyl)-L-tyrosin(O-methyl)-N-(im)-trityl-L-histidin (1-S-cyclohexylmethyl-2-S-hydroxy-5-(2-pyridyl))-pentylamid

Die Titelverbindung wurde aus N-(N-Tert-butoxycarbonyl-cis-4-aminocyclohexylcarbonyl)-L-tyrosin(O-methyl) und L-His(trityl)-(1-S-Cyclohexylmethyl-2-S-hydroxy-5-(2-pyridyl))-pentylamid nach dem in Beispiel 1 beschriebenen Verfahren hergestellt. $R_F$0,75 (LM2)
MS (FAB) 1058 (M+1)

**Beispiel 15**

N-(N-Tert-butoxycarbonyl-cis-4-aminocyclohexylcarbonyl)-L-tyrosin(O-methyl)-L-histidin (1-S-cyclohexylmethyl-2-S-hydroxy-5-(2-pyridyl))-pentylamid

Die Titelverbindung wurde nach dem in Beispiel 6 beschriebenen Verfahren aus der in Beispiel 14 beschriebenen Verbindung hergestellt. $R_F$0,80 (LM 4)
MS (FAB) 816 (M+1)

**Beispiel 16**

N-(cis-4-Aminocyclohexylcarbonyl)-L-tyrosin(O-methyl)-L-histidin (1-S-cyclohexylmethyl-2-S-hydroxy-5-(2-pyridyl))-pentylamid

Die Titelverbindung wurde nach dem in Beispiel 7 beschriebenen Verfahrne aus der in Beispiel 14 beschriebenen Verbindung hergestellt. $R_F$0,50 (LM 4)
MS (FAB) 716 (M+1).

**Beispiel 17**

N-(N-(N-(cis-4-Tert.butoxycarbonylamino-cyclohexylcarbonyl-phenylalaninyl)-histidinyl)-2-(5-S-amino-6-cyclohexyl-3S, 4R-dihydroxy-n-hexyl)-pyridin

60 mg der Verbindung aus Beispiel 17 a) werden mit 40 mg Thiophenol in 2 ml Acetonitril 2 h gerührt. Nach Einengen wird chromatographiert (Kieselgel, LM4) und man erhält 45 mg als amorphes Pulver.
MS (FAB) 802 (M+1)

a) Boc-cis-ACC-Phe-His(DNP)-2-(5-amino-6-cyclohexyl-3S,4R-dihydroxy-n-hexyl)-pyridin

60 mg BOC-His(DNP)-(3S,4R,5S)-2-(5-amino-6-cyclohexyl-3,4-dihydroxy-hexyl)-pyridin werden in 5 ml DME/HCl 2 h gerührt. Nach Einengen wird das Rohprodukt zusammen mit 50 mg N-(N-tert.butoxycarbonyl-cis-4-aminocyclohexylcarbonyl)-L-phenylalanin, 120 mg DCC und 80 mg HOBt in 3 ml DMF gelöst. Der pH-Wert der Lösung wird mit NEM auf pH 9 gestellt und 16 h gerührt. Die Lösung wird filtriert, mit Essigester verdünnt und je einmal mit 3 %iger $NaHCO_3$-Lösung, $H_2O$ und ges. NaCl-Lösung gewaschen, mit $MgSO_4$ getrocknet, eingeengt und an Kieselgel (LM3) chromatographiert. Man erhält 60 mg der Titelverbindung als gelben Schaum.
MS (FAB) 968 (M+1)

b) BOC-His(DNP)-(3S,4R,5S)-2-(5-amino-6-cyclohexyl-3,4-dihydroxy-hexyl)-pyridin

0,5 mmol des (3S,4R,5S)-Isomeren aus Beispiel 17 c werden mit 5 ml HCl in DME (gesättigt) 2 h gerührt. Nach Einengen in Vakuum wird der Rückstand in 3 ml absolutem DMF gelöst. Je 0,5 mmol BOC-His(DNP)-OH, Dicyclohexylcarbodiimd und HOBt werden zugegeben. Die Lösung wird mit NEM auf pH 9 gestellt und 24 h gerührt. Nach Filtration wird mit EE verdünnt und je 3 %iger $NaHCO_3$-Lösung gewaschen, mit $MgSO_4$ getrocknet und eingeengt. Chromatographie an Kieselgel (LM1) ergibt die Titelverbindung als gelbes Harz.
MS (FAB) 696 (M+1)

c) 2-((3S,4R,5S)-5-tert.butoxycarbonylamino-3,4-dihydroxy-6-cyclohexyl-n-hexyl)-pyridin

93 mg (1 mmol) 2-Picolin in 10 ml THF werden bei -78° C mit 1,4 ml (1 mmol) n-BuLi versetzt. Nach Aufwärmen auf RT wird 30 min gerührt, dann auf -40° C abgekühlt. 1 mmol (2RS,3R,4S)-3-tert.-Butyldimethyl-silyloxy-4-tert.-butoxycarbonylamino-5-cyclohexyl-1,2-oxopentan (bekannt aus EP-A 189 203, Beispiel 6) werden zugegeben (gelöst in 5 ml THF). Nach 10 Stunden bei RT wurd mit Wasser verdünnt und mit MTBE extrahiert. Das Rohprodukt (0,4 g) wird in THF gelöst und mit 5 ml einer 1M Lösung von Tetrabutylammoniumfluorid in THF 1 h bei 0° C gerührt. Nach Verdünnen mit Wasesr und Extrahieren mit EE erhält man
0,15 g des (3S,4R,5S)-Isomeren (MS (FAB) : 391 (M+1) und
0,12 g des (3S,4S,5S)-Isomeren (MS (FAB) : 391 (M+1).

**Beispiel 18**

cis-4-Aminocyclohexylcarbonyl-L-Phe-L-His-(1-S-cyclohexylmethyl-2R,3S-dihydroxy-5-(2-pyridyl))-n-pentylamid-tris-trifluoracetat

25 mg der in Beispiel 17 beschriebenen Verbindung werden in 1 ml TFA/ 1 ml $CH_2Cl_2$ 2 h gerührt. Nach Einengen wird in $H_2O$ aufgenommen und lyophilisiert. Man erhält 20 mg Produkt als farbloses Pulver.
MS (FAB) 702 (M+1)

**Beispiel 19**

BOC-trans-ACC-L-Phe-L-His-(1S-cyclohexylmethyl-2R,3S-dihydroxy-5-(2-pyridyl))-n-pentylamid

Die Titelverbindung wurde nach dem in Beipiel 17 beschriebenen Verfahren aus der im Beispiel 19a beschriebenen Verbindung hergestellt. $R_F$ 0,19 (LM 2). Ausbeute 79,5mg
MS (FAB) 802 (M+1)

a) BOC-trans-ACC-L-Phe-L-His(DNP)-(1S-cyclohexylmethyl-2R,3S-dihydroxy-5-(2-pyridyl)-n-pentylamid

Die Titelverbindung wurde nach dem in Beispiel 17 a) beschriebenen Verfahren aus den in den Beispielen 3 a) und 19 b) beschriebenen Verbindungen hergestellt. $R_F$ 0,40 (LM 2)
MS (FAB) 968 (M+1)

b) H-His(DNP)-(3S,4R,5S)-2-(5-amino-6-cyclohexyl-3,4-dihydroxy-n-hexyl)-pyridin-hydrochlorid

400 mg der Verbindung aus Beispiel 17 b) werden in 10 ml DME/HCl (ges.) 2 h gerührt. Nach Einengen wird zweimal in Toluol aufgenommen und jeweils erneut eingeengt. Man erhält die Titelverbindung als gelben Schaum.
MS (FAB) 596 (M+1)

**Beispiel 20**

trans-ACC-L-Phe-L-His-(1S-cyclohexylmethyl-2R,3S-dihydroxy-5-(2-pyridyl))-n-pentylamid

Die Titelverbindung wurde nach dem in Beispiel 18 beschriebenen Verfahren aus der im Beispiel 19 beschriebenen Verbindung hergestellt. Die Ausbeute betrug 49 mg.
MS (FAB) 702 (M+1)

**Beispiel 21**

BOC-trans-ACC-L-Tyr(O-Methyl)-L-His-(1S-cyclohexylmethyl-2R,3S-dihydroxy-5-(2-pyridyl))-n-pentylamid

Die Titelverbindung wurde nach dem in Beispiel 17 beschriebenen Verfahren aus der im Beispiel 21 a) beschriebenen Verbindung hergestellt.
MS (FAB) 832 (M+1)

a) BOC-trans-ACC-L-Tyr(O-Methyl)-L-His(DNP)-(1S-cyclohexylmethyl-2R,3S-dihydroxy-5-(2-pyridyl))-n-pentylamid

Die Titelverbindung wurde nach dem in Beispiel 17 a) beschriebenen Verfahren aus den in den Beispielen 21 b) und 19 b) beschriebenen Verbindungen hergestellt.
MS (FAB) 998 (M+1)

b) BOC-trans-ACC-Tyr($OCH_3$)-OH

0,05 mol BOC-trans-ACC-Tyr($OCH_3$)-$OCH_3$ werden in 250 ml DME gelöst, mti equimolarer Menge an 1N-NaOH-Lösung 5 h gerührt. Danach wird die Lösung eingeengt, der Rückstand mit Wasser aufgenommen, mit $KHSO_4$ auf pH 4 angesäuert und mit EE extrahiert. Das Rohprodukt wird aus n-Heptan/EE umkristallisiert und die Titelverbindung als weißes Pulver erhalten. Schmp. 181 °C Zers. $R_F$ 0.20 (Toluol/Ethanol 8:2) MS (DCI) 421 (M+1)

c) BOC trans-ACC-Tyr($OCH_3$)-$OCH_3$

0,1 mol BOC-trans-ACC-OH und 0,1 mol L-Tyrosin($OCH_3$)-methylester werden in 500 ml DMF gelöst und bei 0 °C utner $N_2$ mit 100 ml n-PropPA (50 %ig in $CH_2Cl_2$) und 0,5 mol Triethylamin verrührt. Die Lösung wird über Nacht zur Reaktion gebracht. Nach beendeter Reaktion (DC-Kontrolle) wird das Lösungsmittel im Hochvakuum abdestilliert und der Rückstand in EE aufgenommen. Die organische Phase wird jeweils zweimal mit 10 %iger Citronensäure, Wasser, halbges. $NaHCO_3$-Lösung und ges. NaCl-Lösung extrahiert, über $MgSO_4$ getrocknet, i.Vak. eingeengt und chromatographiert (Kieselgel, LM5). Schmp. 150-155 °C
MS (DCI) 435 (M+1)

**Beispiel 22**

trans-ACC-L-Tyr(O-Methyl)-L-His-(1S-cyclohexylmethyl-2R,3S-dihydroxy-5-(2-pyridyl))-n-pentylamid

Die Titelverbindung wurde nach dem in Beispiel 18 beschriebenen Verfahren aus der im Beispiel 21 beschriebenen Verbindung hergestellt.
MS (FAB) 732 (M+1)

**Beispiel 23**

BOC-cis-ACC-L-Tyr(O-Methyl)-L-His-(1S-cyclohexylmethyl-2R,3S-dihydroxy-5-(2-pyridyl))-n-pentylamid

Die Titelverbindung wurde nach dem in Beispiel 17 beschriebenen Verfahren aus der im Beispiel 23 a) beschriebenen Verbindung hergestellt. $R_F$ 0,21 (LM 2)
MS (FAB) 832 (M+1)

a) BOC-cis-ACC-L-Tyr(O-Methyl)-L-His(DNP)-(1S-cyclohexylmethyl-2R,3S-dihydroxy-5-(2-pyridyl))-n-pentylamid

Die Titelverbindung wurde nach dem in Beispiel 17 a) beschriebenen Verfahren aus den in den Beispielen 23 b) und 19 b) beschriebenen Verbindungen hergestellt.
MS (FAB 998 (M+1)

b) BOC-cis-ACC-Tyr($OCH_3$)-OH

Die Titelverbindung wird analog dem Verfahren des Beispiels 21b) erhalten. $\alpha_D^{25}$ = + 18,6 ° (c=1, Methanol)
MS (DCI) 421 (M+1)

c) BOC-cis-ACC-Tyr($OCH_3$)-methylester

Die Titelverbindung wurde analog dem Verfahren in Beispiel 21 c) hergestellt.
MS (DCI) 435 (M+1)

**Beispiel 24**

cis-ACC-L-Tyr(O-Methyl)-L-His-(1S-cyclohexylmethyl-2R,3S-dihydroxy-5-(2-pyridyl))-n-pentylamid

Die Titelverbindung wurde nach dem in Beispiel 18 beschriebenen Verfahren aus der im Beispiel 23 beschriebenen Verbindung hergestellt. $R_F$ 0,56 (LM 4)
MS (FAB) 732 (M+1)

**Beispiel 25**

BOC-cis-ACC-Phe-His-(1S-cyclohexylmethyl-2S-hydroxy-5-(N-propyl-2-imidazolyl))-pentylamid

Die Titelverbindung wurde nach dem in Beispiel 17 beschriebenen Verfahren aus der Verbindung in Beispiel 25 hergestellt.
MS (FAB) 817 (M+1)

a) BOC-cis-ACC-Phe-His(DNP)-(1S-cyclohexylmethyl-2S-hydroxy-5-(N-propyl-2-imidazolyl))-pentylamid

80 mg der Verbindung aus Beispiel b werden mit 4 ml HCl in DME (ges.) 2 h gerührt. Nach Einengen im Vakuum wird der Rückstand in 3 ml DMF absolut gelöst. 44 mg BOC-cis-ACC-Phe-OH und je 0,11 mmol DCC und HOBt werden zugegeben. Die Lösung wird mit NEM auf pH 9 gestellt und 24 h gerührt. Nach Filtration wird mit EE verdünnt und je zweimal mit 3 %iger $NaCO_3$-Lösung $H_2O$ und ges. NaCl-Lösung gewaschen, mit $MgSO_4$ getrocknet und eingeengt. Chromatographie an Kieselgel ergibt die Titelverbindung als gelbes Harz.
MS (FAB) 983 (M + 1)

b) BOC-His(DNP)-(1S-cyclohexylmethyl-2S-hydroxy-5-(N-propyl-2--imidazolyl))-pentylamid

100 mg der Verbindung aus Beispiel c werden mit 5 ml HCl in DME (ges.) 2 h gerührt. Nach Einengen im Vakuum wird der Rückstand in 3 ml DMF absolut gelöst. 105 mg BOC-His(DNP)-OH und je 0,3 mmol DCC und HOBt werden zugegeben. Die Lösung wird mit NEM auf pH 9 gestellt und 24 h gerührt. Nach Filtration wird mit EE verdünnt und je zweimal mit 3 %iger $NaCO_3$-Lösung, $H_2O$ und ges. NaCl-Lösung gewaschen, mit $MgSO_4$ getrocknet und eingeengt. Chromatographie an Kieselgel ergibt die Titelverbindung als gelbes Harz.
MS(FAB) 711 (M + 1)

c) 3-BOC-4S-cyclohexylmethyl-2,2-dimethyl-5-(3-(2-N-propylimidazolyl)-propyl)oxazolidin

500 mg der Verbindung aus Beispiel d werden in 5 ml THF absolut gelöst. Unter Argonatmosphäre gibt man bei -60 °C 1,9 ml einer 1,5 molaren Lösung von n-Butyl-Lithium in n-Hexan zu. Nach 15 min. addiert man 500 mg der Verbindung aus Beispiel e gelöst in 5 ml THF absolut. Nach 1 h bei -60 °C gibt man 10 ml ges. $NaHCO_3$-Lösung zu. Nach Aufwärmen auf RT wird dreimal mit EE extrahiert, mit $Na_2SO_4$ getrocknet und eingeengt. Die Titelverbindung erhält man durch Chromatographie an Kieselgel (LM EE).
R(f) 0,3 (EE); MS (FAB) 408 (M + 1)

d) 2-Methyl-N-n-propyl-imidazol

10 g 2-Methyl-imidazol werden in 40 ml n-Propylbromid 2 h zum Sieden erhitzt. Anschließend wird auf 150 ml 5 %ige $NaHSO_4$-Lösung gegossen, dreimal mit EE extrahiert, mit $Na_2SO_4$ getrocknet und eingeengt. Der Rückstand wird in Ether aufgenommen und von unlöslichem Ausgangsmaterial durch Filtration abgetrennt. Auf diese Wiese fällt die Titelverbindung als farbloses Öl an.
R(f) 0,2 ($EE/CH_3OH$ 10:1)

e) 3-Boc-cyclohexylmethyl-2,2-dimethyl-5-(2-bromethyl)-oxazolidin

Zu 690 mg 3-BOC-1S-cyclohexylmethyl-2,2-dimethyl-5-(2-hydroxyethyl)-oxazolidin, 2,6 g Triphenylphosphin und 1,6 g Pyridiniumbromid in 15 ml $CH_2Cl_2$ werden unter Argon 1,6 ml Azodicarbonsäurediethylester bei 20 °C zugetropft. Nach 16 h bei RT wird Wasser zugegeben und mit 100 ml $CH_2Cl_2$ verdünnt. Die organische Phase wird zweimal mit ges. $NaHCO_3$-Lösung und einmal mit NaCl-Lösung gewaschen. Die mit $Na_2SO_4$ getrocknete organische Phase wird eingeengt, der Rückstand in wenig EE aufgenommen und zur Abtrennung von $PPh_3$ filtriert. Reinigung an Kieselgel liefert die Titelverbindung (LM6).
R(f) 0,3 (LM6); MS 404 (M + 1).

f) 3-BOC-4S-cyclohexylmethyl-2,2-dimethyl-5-(2-hydroxyethyl)-oxazolidin

10 g BOC-ACHPA-$OC_2H_5$ (Herstellung nach J. Med. Chem. 28, 1779 (1985)), 500 mg p-Toluolsulfonsäure und 7,2 ml Dimethoxypropan werden in 160 ml Toluol unter Argon 2 h auf 80 °C erhitzt. Anschließend wird eingeengt. Der Rückstand wird bei 0 °C zu einer Suspension aus 2 g $LiALH_4$ in 200 ml THF getropft. Nach 2,5 h bei 0 °C werden 100 ml 5 %ige $NaHSO_4$-Lösung zugegeben und dreimal mit EE extrahiert. Die

vereinigten organischen Phasen werden mit ges. $NaHCO_3$-Lösung gewaschen. Nach dem Trocknen mit $Na_2SO_4$ wird eingeengt und über Kieselgel chromatographiert (LM6).
R(f) 0,1 (LM6); MS (DCI) 342 (M+1).

### Beispiel 26

cis-ACC-Phe-His-(1S-cyclohexylmethyl-2S-hydroxy-5-(N-propyl-2-imidazolyl))-pentylamid

Die Titelverbindung wurde nach dem in Beispiel 18 beschriebenen Verfahren aus der Verbindung in Beispiel 25 hergestellt.
MS (FAB) 717 (M+1).

## Ansprüche

1. Verbindungen der Formel I

$$R^a\text{-}W\text{-}A\text{-}B\text{-}NH\text{-}\overset{\overset{R^2}{|}}{CH}\text{-}\overset{\overset{OH}{|}}{CH}\text{-}\overset{\overset{R^3}{|}}{CH}\text{-}R^4 \qquad (I)$$

in welcher
$R^a$ $(C_3\text{-}C_8)$-Cycloalkyl, $(C_4\text{-}C_{10})$-Bicycloalkyl, $(C_8\text{-}C_{12})$-Tricycloalkyl, $(C_3\text{-}C_8)$-Cycloalkyl-$(C_1\text{-}C_6)$-Alkyl, $(C_4\text{-}C_{10})$-Bicycloalkyl-$(C_1\text{-}C_6)$-alkyl und $(C_8\text{-}C_{12})$-Tricycloalkyl-$(C_1\text{-}C_6)$-alkyl bedeutet, worin jeweils der Cycloalkyl-, Bicycloalkyl- und Tricycloalkyl-Substituent durch einen oder zwei gleiche oder verschiedene Reste aus der Reihe F, Cl, Br, I, Hydroxy, $(C_1\text{-}C_6)$-Alkoxy, $(C_1\text{-}C_6)$-Alkyl, Carboxy, $(C_1\text{-}C_6)$-Alkoxycarbonyl, Carbamoyl, Carboxymethoxy, Amino, $(C_1\text{-}C_6)$-Monoalkylamino, $(C_1\text{-}C_6)$-Dialkylamino, Amino-$(C_1\text{-}C_6)$-Alkyl, $(C_1\text{-}C_6)$-Alkylamino-$(C_1\text{-}C_6)$Alkyl, Di-$(C_1\text{-}C_6)$-Alkylamino-$(C_1\text{-}C_6)$-Alkyl, Amidino, Hydroxamino, Hydroximino, Hydrazono, Imino, Guanidino, $(C_1\text{-}C_6)$-Alkyloxysulfonyl, $(C_1\text{-}C_6)$-Alkyloxysulfenyl, Trifluormethyl, $(C_1\text{-}C_4)$-Alkoxycarbonylamino, $(C_6\text{-}C_{12})$-Aryl-$(C_1\text{-}C_4)$-Alkoxycarbonylamino, substituiert sein kann;
W für -CO-, -O-CO-, $-SO_2-$ steht
$R^2$ Wasserstoff, $(C_1\text{-}C_{10})$-Alkyl, $(C_4\text{-}C_7)$-Cycloalkyl, $(C_4\text{-}C_7)$-Cycloalkyl-$(C_1\text{-}C_4)$-Alkyl, $(C_6\text{-}C_{14})$-Aryl oder $(C_6\text{-}C_{14})$-Aryl-$(C_1\text{-}C_4)$Alkyl bedeutet,
$R^3$ Wasserstoff, $(C_1\text{-}C_{10})$-Alkyl, $(C_6\text{-}C_{14})$-Aryl, $(C_6\text{-}C_{14})$-Aryl-$(C_1\text{-}C_4)$-Alkyl, Hydroxy oder Amino bedeutet,
$R^4$ einen Rest der Formel II bedeutet,
$(CH_2)_m\text{-}CHR^5\text{-}D$ (II)
wobei
$R^5$ für Wasserstoff, $(C_1\text{-}C_7)$-Alkyl, $(C_1\text{-}C_5)$-Alkoxy, $(C_1\text{-}C_5)$-Alkylthio, $(C_1\text{-}C_5)$-Alkylamino, Hydroxy, Azido, Fluor, Chlor, Brom, Jod steht,
D einen Rest Het bedeutet, wobei
Het einen 5- bis 7-gliedrigen heterocyclischen Ring steht, der benzannelliert, aromatisch, teilhydriert oder vollständig hydriert sein kann, der als Heteroatome einen der zwei gleiche oder verschiedene Reste aus der Gruppe N, O, S, NO, SO, $SO_2$ enthalten kann und der durch einen oder zwei gleiche oder verschiedene Reste aus der Gruppe $(C_1\text{-}C_4)$-Alkyl, $(C_1\text{-}C_4)$-Alkoxy, Hydroxy, Halogen, Amino, Mono- oder Di-$(C_1\text{-}C_4)$-alkylamino, $CF_3$ substituiert sein kann, und
m 0, 1, 2, 3 oder 4 bedeutet;
A und B unabhängig voneinander einen N-terminal mit $R^a$-W bzw. A und C-terminal mit B bzw. $NH\text{-}CHR^2\text{-}CHOH\text{-}CHR^3\text{-}R^4$ verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin,4-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]-thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, N-Methyl-Histidin, 2-Amino-4-(2-thienyl)-buttersäure, 2-Amino- 4-(3-thienyl)-buttersäure, 3-(2-Thienyl)-serin, (Z)-Dehydrophenylalanin, (E)-Dehydrophenylalanin, 1,3-Dioxolan-2-yl-alanin, N-Pyrrolylalanin und 1-, 3-oder 4-Pyrazolylalanin bedeuten,
sowie deren physiologisch verträgliche Salze.

2. Verbindungen gemäß Anspruch 1, dadurch gekennzeichnet, daß $R^a$ und W wie in Anspruch 1 definiert sind,
$R^2$ Wasserstoff, $(C_1-C_{10})$-Alkyl, $(C_4-C_7)$-Cycloalkyl, $(C_4-C_7)$-Cycloalkyl-$(C_1-C_4)$-Alkyl, $(C_6-C_{14})$-Aryl oder $(C_6-C_{14})$-Aryl, $(C_1-C_4)$Alkyl bedeutet,
$R^3$ Wasserstoff, $(C_1-C_{10})$-Alkyl, $(C_6-C_{14})$-Aryl, $(C_6-C_{14})$-Aryl-$(C_1-C_4)$-Alkyl, Hydroxy oder Amino bedeutet,
$R^4$ einen Rest der Formel II bedeutet, worin
$R^5$ für Wasserstoff, $(C_1-C_4)$-Alkyl, $(C_1-C_4)$-Alkoxy, $(C_1-C_4)$-Alkylthio, $(C_1-C_4)$-Alkylamino, Hydroxy, Azido, Fluor, Chlor, Brom oder Jod steht,
D wie der Rest Het im Anspruch 1 definiert ist, und m 0, 1 oder 2 bedeutet; und A und B unabhängig voneinander einen bivalenten Rest aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 4-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, N-Methyl- Histidin, 2-Amino-4-(2-thienyl)-buttersäure, 2-Amino-4-(3-thienyl)-buttersäure, 3-(2-Thienyl)-serin, (Z)-Dehydrophenylalanin, (E)-Dehydrophenylalanin, 1,3-Dioxolan-2-yl-alanin, N-Pyrrolylalanin und 1-, 3-oder 4-Pyrazolylalanin bedeuten,
sowie deren physiologisch verträgliche Salze.

3. Verbindungen gemäß Anspruch 1 und/oder 2, dadurch gekennzeichnet, daß
$R^a$ $(C_4-C_6)$-Cycloalkyl und $(C_4-C_6)$-cylcoalkyl-$(C_1-C_2)$-Alkyl bedeutet, worin jeweils der Cycloalkyl-Substituent durch einen oder zwei gleiche oder verschiedene Reste aus der Reihe Hydroxy, $(C_1-C_4)$-Alkoxy, $(C_1-C_2)$-Alkyl, Carboxy, $(C_1-C_2)$-Alkoxycarbonyl,Carbamoyl, Carboxymethoxy, Amino, $(C_1-C_2)$-Monoalkylamino, $(C_1-C_2)$-Dialkylamino, Amino-$(C_1-C_2)$-Alkyl, $(C_1-C_2)$-Alkylamino-$(C_1-C_2)$Alkyl, Di-$(C_1-C_2)$-Alkylamino-$(C_1-C_2)$-Alkyl, Amidino, Trifluormethyl, $(C_1-C_4)$-Alkoxycarbonylamino, wie tert.Butoxycarbonylamino, $(C_6-C_{12})$-Aryl-$(C_1-C_2)$-Alkoxy-Carbonylamino wie Benzyloxycarbonylamino und Methansulfonylamido, Trifluormethansulfonylamido substituiert sein kann;
W für -CO- steht
$R^2$ Isobutyl, Benzyl oder Cyclohexylmethyl bedeutet;
$R^3$ Wasserstoff oder Hydroxy bedeutet;
$R^4$ einen Rest der Formel II bedeutet, in welcher
$R^5$ für Wasserstoff oder Fluor steht,
D für einen 2-, 3- oder 4-Pyridinrest, einen 2-, 4-oder 5-Imidazolrest oder einen 2-Oxazolinrest steht, wobei die genannten Heterocyclen jeweils durch einen oder zwei Reste aus der Gruppe Methyl, Methoxy,Ethyl, Propyl, Allyl, Fluor, Chlor, Brom und $CF_3$ substituiert sein können, und
m 0, 1 oder 2 bedeutet; und
A und B unabhängig voneinander einen bivalenten Rest aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin,4-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, 1,2,3,4-Tetrahydroisochinolin-3-carbonsäure, Homophenylalanin, 2-Amino-4-(2-thienyl)-buttersäure und 1-, 3- und 4-Pyrazolylalanin bedeuten,
sowie deren physiologisch verträgliche Salze.

4. Verfahren zur Herstellung einer Verbindung der Formel I, gemäß einem oder mehreren der Ansprüche 1 bis 3, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

5. Verbindung der Formel I gemäß einem oder mehreren der Ansprüche 1 bis 3 zur Anwendung als Heilmittel.

6. Verbindung der Formel I gemäß einem oder mehreren der Ansprüche 1 bis 3 zur Anwendung als

Heilmittel bei der Behandlung des Bluthochdrucks.

7. Pharmazeutische Zubereitung enthaltend eine Verbindung der Formel I gemäß einem oder mehreren der Ansprüche 1 bis 3.

Patentansprüche für folgende Vertragsstaaten : ES, GR

1. Verfahren zur Herstellung einer Verbindung der Formel I

$$R^a\text{-}W\text{-}A\text{-}B\text{-}NH\text{-}\underset{\displaystyle |}{\overset{R^2}{}}\!CH\text{-}\overset{OH}{CH}\text{-}\overset{R^3}{CH}\text{-}R^4 \qquad (I)$$

in welcher

$R^a$ ($C_3$-$C_8$)-Cycloalkyl, ($C_4$-$C_{10}$)-Bicycloalkyl, ($C_8$-$C_{12}$)-Tricycloalkyl, ($C_3$-$C_8$)-Cycloalkyl-($C_1$-$C_6$)-Alkyl, ($C_4$-$C_{10}$)-Bicycloalkyl-($C_1$-$C_6$)-alkyl und ($C_8$-$C_{12}$)-Tricycloalkyl-($C_1$-$C_6$)-alkyl bedeutet, worin jeweils der Cycloalkyl-, Bicycloalkyl- und Tricycloalkyl-Substituent durch einen oder zwei gleiche oder verschiedene Reste aus der Reihe F, Cl, Br, I, Hydroxy, ($C_1$-$C_6$)-Alkoxy, ($C_1$-$C_6$)-Alkyl, Carboxy, ($C_1$-$C_6$)-Alkoxycarbonyl, Carbamoyl, Carboxymethoxy, Amino, ($C_1$-$C_6$)-Monoalkylamino, ($C_1$-$C_6$)-Dialkylamino, Amino-($C_1$-$C_6$)-Alkyl, ($C_1$-$C_6$)-Alkylamino-($C_1$-$C_6$)Alkyl, Di-($C_1$-$C_6$)-Alkylamino-($C_1$-$C_6$)-Alkyl, Amidino, Hydroxamino, Hydroximino, Hydrazono, Imino, Guanidino, ($C_1$-$C_6$)-Alkyloxysulfonyl, ($C_1$-$C_6$)-Alkyloxysulfenyl, Trifluormethyl, ($C_1$-$C_4$)-Alkoxycarbonylamino, ($C_6$-$C_{12}$)-Aryl-($C_1$-$C_4$)-Alkoxycarbonylamino, substituiert sein kann;

W für -CO-, -O-CO-, $-SO_2-$ steht

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-Alkyl, ($C_6$-$C_{14}$)-Aryl oder ($C_6$-$C_{14}$)-Aryl-($C_1$-$C_4$)Alkyl bedeutet,

$R^3$ Wasserstoff, ($C_1$-$C_{10}$)-Alkyl, ($C_6$-$C_{14}$)-Aryl, ($C_6$-$C_{14}$)-Aryl-($C_1$-$C_4$)-Alkyl, Hydroxy oder Amino bedeutet,

$R^4$ einen Rest der Formel II bedeutet,

$(CH_2)_m$-$CHR^5$-D (II)

wobei

$R^5$ für Wasserstoff, ($C_1$-$C_7$)-Alkyl, ($C_1$-$C_5$)-Alkoxy, ($C_1$-$C_5$)-Alkylthio, ($C_1$-$C_5$)-Alkylamino, Hydroxy, Azido, Fluor, Chlor, Brom, Jod steht,

D einen Rest Het bedeutet, wobei

Het einen 5- bis 7-gliedrigen heterocyclischen Ring steht, der benzannelliert, aromatisch, teilhydriert oder vollständig hydriert sein kann, der als Heteroatome einen oder zwei gleiche oder verschiedene Reste aus der Gruppe N, O, S, NO, SO, $SO_2$ enthalten kann und der durch einen oder zwei gleiche oder verschiedene Reste aus der Gruppe ($C_1$-$C_4$)-Alkyl, ($C_1$-$C_4$)-Alkoxy, Hydroxy, Halogen, Amino, Mono- oder Di-($C_1$-$C_4$)-alkylamino, $CF_3$ substituiert sein kann, und

m 0, 1, 2, 3 oder 4 bedeutet;

A und B unabhängig voneinander einen N-terminal mit $R^1$ bzw. A und C-terminal mit B bzw. NH-$CHR^2$-CHOH-$CHR^3$-$R^4$ verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 4-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2,4-Naphthylalanin, 4-Nitrophenylalanin, Norvalin, β-2-Benzo[b]thienylalanin, β-3-Benzo[b]-thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, N-Methyl-Histidin, 2-Amino-4-(2-thienyl)-buttersäure, 2-Amino- 4-(3-thienyl)-buttersäure, 3-(2-Thienyl)-serin, (Z)-Dehydrophenylalanin, (E)-Dehydrophenylalanin, 1,3-Dioxolan-2-yl-alanin, N-pyrrolylalanin und 1-, 3-oder 4-Pyrazolylalanin bedeuten,

sowie deren physiologisch verträgliche Salze, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren gemäß Anspruch 1, dadurch gekennzeichnet, daß $R^a$ und W wie in Anspruch 1 definiert sind,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-Alkyl, ($C_6$-$C_{14}$)-Aryl oder ($C_6$-

$C_{14}$)-Aryl, ($C_1$-$C_4$)-Alkyl bedeutet,
$R^3$ Wasserstoff, ($C_1$-$C_{10}$)-Alkyl, ($C_6$-$C_{14}$)-Aryl, ($C_6$-$C_{14}$)-Aryl-($C_1$-$C_4$)-Alkyl, Hydroxy oder Amino bedeutet,
$R^4$ einen Rest der Formel II bedeutet, worin
$R^5$ für Wasserstoff, ($C_1$-$C_4$)-Alkyl, ($C_1$-$C_4$)-Alkoxy, ($C_1$-$C_4$)-Alkylthio, ($C_1$-$C_4$)-Alkylamino, Hydroxy, Azido, Fluor, Chlor, Brom oder Jod steht,
D wie der Rest Het auf Seite 2 definiert ist, und Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 4-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, N-methyl-Histidin, 2-Amino-4-(2-thienyl)-buttersäure, 2-Amino-4-(3-thienyl)-buttersäure, 3-(2-Thienyl)-serin, (Z)-Dehydrophenylalanin, (E)-Dehydrophenylalanin, 1,3-Dioxolan-2-yl-alanin, N Pyrrolylalanin und 1-, 3-oder 4-pyrazolylalanin bedeuten,

3. Verfahren gemäß Anspruch 1 und/oder 2, dadurch gekennzeichnet, daß
$R^a$ ($C_4$-$C_6$)-Cycloalkyl und ($C_4$-$C_6$)-Cylcoalkyl-($C_1$-$C_2$)-Alkyl bedeutet, worin jeweils der Cycloalkyl-Substituent durch einen oder zwei gleiche oder verschiedene Reste aus der Reihe Hydroxy, ($C_1$-$C_4$)-Alkoxy, ($C_1$-$C_2$)-Alkyl, Carboxy, ($C_1$-$C_2$)-Alkoxycarbonyl,Carbamoyl, Carboxymethoxy, Amino, ($C_1$-$C_2$)-Monoalkylamino, ($C_1$-$C_2$)-Dialkylamino, Amino-($C_1$-$C_2$)-Alkyl, ($C_1$-$C_2$)-Alkylamino-($C_1$-$C_2$)-Alkyl, Di-($C_1$-$C_2$)-Alkylamino-($C_1$-$C_2$)-Alkyl, Amidino, Trifluormethyl, ($C_1$-$C_4$)-Alkoxycarbonylamino, wie tert.Butoxycarbonylamino, ($C_6$-$C_{12}$)-Aryl-($C_1$-$C_2$)-Alkoxy-Carbonylamino wie Benzyloxycarbonylamino und Methansulfonylamido, Trifluormethansulfonylamido substituiert sein kann;
W für -CO- steht
$R^2$ Isobutyl, Benzyl oder Cyclohexylmethyl bedeutet;
$R^3$ Wasserstoff oder Hydroxy bedeutet;
$R^4$ einen Rest der Formel II bedeutet, in welcher
$R^5$ für Wasserstoff oder Fluor steht,
D für einen 2-, 3- oder 4-Pyridinrest, einen 2-, 4-oder 5-Imidazolrest oder einen 2-Oxazolinrest steht, wobei die genannten Heterocyclen jeweils durch einen oder zwei Reste aus der Gruppe Methyl, Methoxy, Fluor, Chlor, Brom und $CF_3$ substituiert sein können, und
m 0, 1 oder 2 bedeutet; und
A und B unabhängig voneinander einen bivalenten Rest aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, Valin, Alanin, 2,4-Diaminobuttersäure, Arginin, 4-Chlorphenylalanin, Methioninsulfon, Methioninsulfoxid, 2-pyridylalanin, 3-Pyridylalanin, 4-Pyridylalanin, Cyclohexylalanin, Cyclohexylglycin, im-Methylhistidin, O-methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, 1,2,3,4-Tetrahydroisochinolin-3-carbonsäure, Homophenylalanin, 2-Amino-4-(2-thienyl)-buttersäure und 1-, 3- und 4-Pyrazolylalanin bedeuten.

4. Verwendung einer nach einem der Verfahren der Ansprüche 1 bis 3 hergestellten Verbindung der Formel I als Heilmittel.

5. Verwendung einer nach einem der Verfahren der Ansprüche 1 bis 3 hergestellten Verbindung der Formel I bei der Behandlung des Bluthochdrucks.

6. Verfahren zur Herstellung einer pharmazeutischen Zubereitung enthaltend eine nach einem der Verfahren der Ansprüche 1 bis 3 hergestellten Verbindung der Formel I, dadurch gekennzeichnet, daß man diese zusammen mit einem physiologisch unbedenklichen Träger und gegebenenfalls weiteren Hilfs- und Zusatzstoffen in eine geeignete Darreichungsform bringt.

Europäisches Patentamt

# EUROPÄISCHER RECHERCHENBERICHT

Nummer der Anmeldung

EP 90 10 7491

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 255 082 (HOECHST AG)<br>* Ganzes Dokument *<br>--- | 1-7 | C 07 K 5/06<br>A 61 K 37/64 |
| Y | GB-A-2 130 221 (SQUIBB & SONS)<br>* Seite 1, Zeile 6 - Seite 4, Zeile 12; Seite 8, Zeilen 16-21; Beispiele 18-29,35-82,89; Ansprüche 1-32 *<br>--- | 1-7 | |
| A | EP-A-0 231 919 (SQUIBB & SONS)<br>* Seite 3, Zeile 18 - Seite 8, Zeile 17; Seite 20, Zeile 21 - Seite 26, Zeile 1; Beispiele 13-16,19,46; Ansprüche 1-43 *<br>----- | 1-7 | |
| | | | **RECHERCHIERTE SACHGEBIETE (Int. Cl.5)** |
| | | | C 07 K<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 24-07-1990 | GROENENDIJK M.S.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)